# EUROPEAN PATENT APPLICATION

(11) **EP 2 011 471 A1**
(43) Date of publication of application: **07.01.2009**
(21) Application number: 07012898.8
(22) Date of filing: 02.07.2007
(51) Int. Cl.: A61K 8/04, A61K 8/31, A61K 8/34, A61K 8/36, A61K 8/37, A61K 8/58, A61K 8/891, A61K 8/892, A61K 8/893, A61K 8/895, A61K 8/92, A61Q 1/14, A61Q 19/10

(54) **Foaming oil compositions**

(71) Applicant: Johnson & Johnson Consumer France SAS, 92787 Issy les Moulineaux Cedex 9 (FR)
(72) Inventor: Arif, Ambareen, 8002 Zürich (CH); Mutti, Bruna, 40545 Düsseldorf (DE); Rouet, Sophie, Louviers 27400 (FR)
(74) Representative: Metten, Karl-Heinz

(57) **Abstract**

A personal care composition comprising an oil and a siloxane resin foam stabilizer, said composition being substantially free of surfactants and propellants, is provided. The composition is capable of foaming, and is advantageously clear and non-greasy.

## Description

### FIELD OF THE INVENTION

The invention relates to a personal care composition comprising an oil and a siloxane resin foam stabilizer, said composition being substantially free of surfactants and propellants. The composition is capable of foaming using a wide variety of oils and amounts thereof. It is advantageously clear and non-greasy, and ideal for skin care and baby care applications.

### BACKGROUND OF THE INVENTION

oil-containing products as a category of personal care products are often limited in terms of product form due to the need for high amounts of oil and the impact this has on packaging. Typically, both the type and amount of oil is limited. In addition, oil-containing products often have greasy after-feel and may be messy to use. They are also often opaque. However, they are advantageous in terms of providing the consumer with increased perceived softness and greater moisturization.

Delivery of oil-containing products by pump foamers overcomes some of these negative attributes. From a consumer benefit perspective, foaming products are less greasy and messy. Pump foamers also eliminate safety and environmental concerns associated with spray products that use propellants.

EP 1 100 458 81 relates to a foaming oil composition comprising a surfactant, an oil component, and an alkyl phosphate component. However, the presence of surfactants in personal care compositions can lead to skin irritation.

The present invention provides a personal care composition comprising an oil that is substantially free of surfactants and propellants. In spite of this, it foams well and provides a non-greasy, non-messy product. It is mild, clear, and soft, and suited for use in a variety of skin care and baby care applications.

### SUMMARY OF THE INVENTION

This invention relates to a personal care composition comprising an oil and a siloxane resin foam stabilizer, said composition being substantially free of surfactants and propellants and being capable of foaming.

The invention also provides for the use of this personal care composition in a skin care product.

The invention also provides for the use of this personal care composition in a baby care product.

The invention also relates to a method for reducing the greasiness of an oil-containing personal care composition, which comprises adding thereto a siloxane resin foam stabilizer.

### DETAILED DESCRIPTION OF THE INVENTION

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

In one embodiment, the personal care composition comprises an oil and a siloxane resin foam stabilizer, and is substantially free, most preferably free, of surfactants and/or propellants. In preferred embodiments "substantially free" means less than 0.1 weight-%, more preferred less than 0.01 weight-% and in particular preferred less than 0.001 weight-% of surfactants and/or propellants, based on the weight of the personal care composition.

The composition may contain one or more than one oil. The oil may, for example, be selected from the group consisting of natural or synthetic oils from mineral, vegetable or animal sources, fats, hydrocarbon oils, silicone oils, linear volatile polydialkylsiloxanes, in particular linear volatile polydimethylsiloxanes, cyclic volatile polydialkylsiloxanes, in particular cyclic volatile polydimethylsiloxanes, linear nonvolatile polydialkylsiloxanes, in particular linear nonvolatile polydimethylsiloxanes, polyalkyl siloxanes, polyaryl siloxanes, in particular polyphenylsiloxanes, polyalkylaryl siloxane copolymers, in particular polybenzyl siloxane copolymers, silicone resins, siloxy silicate polymers, fatty acid esters, fatty alcohols, fatty acids and mixtures thereof.

Silicone oils useful in the present invention include for example chain silicones such as dimethyl polysiloxane, methyl phenyl polysiloxane, and methyl hydrogen polysiloxane and cyclic silicones such as octamethylcyclotetrasiloxane, decamethylcyclopentasiloxane, and dodecamethylcyclo-hexanesiloxane, and mixtures thereof.

Fats and oils useful in the present invention include, but are not limited to, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, triglycerin, glyceryl trioctanoate, glyceryl triisopalmitate, and mixtures thereof.

Examples of hydrocarbon oils include liquid paraffin, squalane, squalene, paraffin, isoparaffin, ceresine, and the like.

In a preferred embodiment, the oil comprises one or more vegetable oils. Examples of vegetable oils include for example coconut oil, groundnut oil, soybean oil, sunflower oil, olive oil, hydrogenated castor oil, sweet almond oil, calophyllum, palm oil, castor oil, avocado oil, jojoba oil, cereal germ oil, corn oil, cottonseed oil, walnut oil, linseed oil, mink oil, illipe butter, rapeseed oil, soybean oil, sunflower seed oil, blackcurrant oil, borage oil, camelina oil, evening primrose oil, rice bran oil, flaxseed oil, and mixtures thereof.

The siloxane resin is generally non-linear and comprises, in particular consists of, siloxane units of the formula RₐSiO_{4-a/2} wherein R denotes a hydroxyl, hydrocarbon or hydrocarbonoxy group, and wherein a has an average value of from 0.5 to 2.4. It preferably comprises, in particular consists of, monovalent trihydrocarbonsiloxy (M) groups of the formula R"₃SiO_{1/2} and tetrafunctional (Q) groups R"SiO_{4/2} wherein R" denotes a monovalent hydrocarbon group. Such resins can be called MQ resins. The number ratio of M groups to Q groups is preferably in the range from 0.4:1 to 1.2:1 (equivalent to a value of a in the formula RaSi04-a/2 of 0.86 to 1.64) and is preferably a solid at room temperature. Most preferably the ratio of M groups to Q groups is 0.5:1 to 0.8:1 (equivalent to a=1.0 to 1.33). One or more than one siloxane resin may be used.

Although it is most preferred that the siloxane resin comprises only, in particular consists only of, monovalent and tetravalent siloxy units as defined above, a resin comprising M groups, trivalent R"SiO_{3/2} (T) units and Q units can alternatively be used. It is also acceptable that up to 20% of all siloxane units present in the siloxane resin can be divalent units R"₂SiO_{2/2}.

The group R" as used above for the siloxane units is preferably an alkyl group, in particular having 1 to 6 carbon atoms, for example methyl, ethyl, or i- or n-propyl, or can be aryl, in particular phenyl. It is particularly preferred that at least 80%, most preferably substantially all, R" groups present are methyl groups. Other hydrocarbon groups may be present, e.g. alkenyl groups present for example as dimethylvinylsilyl units, preferably not exceeding 5% of all R" groups. Silicon-bonded hydroxyl groups and/or alkoxy, e.g. methoxy, groups may also be present.

Siloxane resins, in particular the preferred MQ resins, are generally insoluble in water-immiscible oils such as mineral oil or vegetable oil. However, such resins are more effective in producing a foam of the desired stability, that is a foam which does not collapse on standing in air but can be broken by continued mechanical action. The siloxane resin is generally used at 0.5 to 30% by weight of the composition, preferably from 1 or 2 up to 20% by weight.

In another embodiment the composition of the invention can further comprise at least one volatile alkane, in particular a C₆- to C₁₈- volatile alkane, or mixtures thereof. Volatile C₁₃₋₁₅ alkanes, or mixtures thereof, are particularly preferred.

Further, in another embodiment the composition of the invention can further comprise at least one solubilizing agent for the siloxane resin foam stabilizer in the form of at least one fatty acid ester. Octyl stearate is particularly preferred.

The personal care composition can be manufactured using preparation and blending techniques conventional in the cosmetics field.

The personal care composition may optionally contain one or more cosmetically active agents or other additives conventional in the skin care or baby care fields. A "cosmetically active agent" is a compound (e.g., a synthetic compound or a compound isolated from a natural source, or a natural extract containing a mixture of compounds) that has a cosmetic or therapeutic effect on the skin, including, but not limiting to, anti-aging agents, lightening agents, darkening agents such as self-tanning agents, anti-acne agents, shine control agents, anti-microbial agents such as anti-yeast agents, anti-fungal agents, and anti-bacterial agents, anti-inflammatory agents, anti-parasite agents, external analgesics, sunscreens, photoprotectors, antioxidants, keratolytic agents, moisturizers, nutrients, vitamins, energy enhancers, anti-perspiration agents, astringents, deodorants, hair removers, hair growth enhancing agents, hair growth delaying agents, firming agents, anti-callous agents, agents for skin conditioning, anti-cellulite agents, fluorides, odor-control agents such as odor masking or pH-changing agents, and actives from natural sources.

The cosmetically active agents are typically present in an amount of from about 0.001% to about 20%, e.g., about 0.005% to about 10%, such as about 0.01% to about 5%, by weight of the composition.

Examples of vitamins include, but are not limited to, vitamin A, vitamin Bs such as vitamin B3, vitamin B5, and vitamin B12, vitamin C, vitamin K, vitamin E such as alpha, gamma, or delta-tocopherol, and derivatives (such as salts and esters) and mixtures thereof.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), different types of tocopherols (e.g., alpha-, gamma-, and delta-tocopherols and their esters such as acetate) and their mixtures, tocotrienols, and ubiquinone. Natural extracts containing antioxidants suitable for use in the compositions of this invention include, but are hot limited to, extracts containing flavonoids, isoflavonoids, and their derivatives such as genistein and diadzein (e.g., such as soy and clover extracts, extracts containing resveratrol and the like). Examples of such natural extracts include grape seed, green tea, pine bark, and propolis.

Examples of enzymes include, but are not limited to, proteases such as fungal proteases, bacterial proteases, or mammalian proteases. Examples of such proteases include, but are not limited to, pepsin, cathepsin, human urinary acid protease, fungal proteases derived from Neurospora oryzae, Mucor pusillus, Mucor miehei, Rhizopus chinensis, or Endothia parasitica, and bacterial proteases rhizopuspepsin, penicillopepsin, and endothiapepsin. Further, the proteases may be derived from processes involving genetic engineering processes and techniques.

Examples of fungal extracts include, but are not limited to, extracts from Neurospora oryzae, Mucor pusillus, Mucor miehei, Rhizopus chinensis, or Endothia parasitica Mucor Miehei. Examples of compositions containing such proteases and fungal extracts are disclosed in U.S. Patent No. 5,976,556.

Examples of natural extracts include, but are not limited to, green tea extract, horse chestnut extract, live yeast cell extract, cucumber extract, aloe vera, feverfew extract, soy extract, boswellia extract, turmeric extract, and derivatives and mixtures thereof. In one embodiment, the plant extract (e.g., a feverfew extract or a soybean extract) is present in the composition in an amount from about 0.001% to about 20% by weight, in particular in an amount from about 0.1% to about 10% by weight of the composition. Unless stated otherwise, the weight of the extract refers to the dry weight of the extract.

Darkening agents such as self-tanning agents include dihydroxy acetone, forskolin, erthulose, skin darkening peptides such as LIGR, SLIGRL, and others disclosed in US Patent Application Publication Nos. 2002/0197219, 2002/0197281, 2003/0138388, and 2003/0232743, melanin and melanin derivatives (e.g., both melanin polymers and lower molecular weight water-soluble melanin derivatives), and extracts of the plant genus Hedychium, Rhubarb, Bearberry, and Onosis such as Onosis Spinosa root extract. Examples of synthetic melanin derivatives are disclosed in U.S. Pat. Nos. 5,618,519, 5,384,116, and 5,227,459 and U.S. Patent Application No. 2005/0129633. Examples of soluble melanin derivatives are disclosed in U.S. Pat. Nos. 5,744,125, 5,225,435, 5,218,079, and 5,216,116. Examples of commercially available soluble melanin derivatives include Melasyn -100^{®} from San-mar laboratories, Inc. (Elmsford, N.Y.) and MelanZe^{®} from Zylepsis (Ashford, Kent, United Kingdom).

In one embodiment, the personal care may contain a surfactant but still be free of propellants. This may be desirable for certain cleansing applications. Nonionic, cationic, anionic, and/or amphoteric surfactants may be used, examples of which are well known in the art. See for example McCutcheon's Detergents and Emulsifiers, North American edition (1986), published by Allured Publishing Corporation; McCutcheon's Functional Materials, North American Edition (1992); and U.S. Pat. No. 3,929,678, to Laughlin et al.

The composition may also contain other additives conventional in skin care and baby care applications, such as emollients, chelating agents (e.g., EDTA), preservatives (e.g., parabens), thickeners, colorants such as dyes and pigments, and the like, or mixtures thereof.

The personal care composition of the present invention has several advantageous properties. It combines good foaming with the benefits of an oil-containing composition. To the consumer, it is mild, is highly moisturizing, absorbent, and clean. It preferably is clear and transparent in physical appearance. It is non-irritating without surfactants, and safe and environmentally friendly without propellants.

It is also non-greasy and non-messy. Accordingly, in one embodiment, a method for reducing the greasiness of an oil-containing personal care composition is provided by this invention. The method comprises adding to such oil-containing personal care composition a siloxane resin foam stabilizer.

The personal care composition may be used for example in skin care products, for instance as an oil bath, shower preparation, face or body cleanser, or make-up remover, particularly an eye make-up remover. The personal care composition may also be useful for treating dry skin conditions and atopic eczema, for example as a pharmaceutical composition.

The personal care composition is also useful in baby care products, especially for its clean, clear, mild, and soft attributes. For example, the composition may be employed in baby care products selected from the group consisting of baby toiletries, baby hair care products, baby skin care products, baby sun care products, baby washes, and baby cleansers.

The following example illustrates the invention without limiting its scope. Unless indicated otherwise, amounts are expressed by weight.

### EXAMPLE

A personal care composition according to the invention was made as follows. Seventy-five percent siloxane resin having trimethyl siloxane units and SiO₂ units in a M/Q ratio of 0.65/1 was dissolved in 25% octyl stearate. Ten percent of the resulting siloxane resin solution was incorporated into an oil-based foamable composition comprising 79.8% GEMSEAL 25 (volatile C13-15 alkane), 0.2% fragrance and 10.0% of a vegetable oil blend to give a transparent, foamable, liquid composition.

The composition was subjected to sensory evaluation for a number of consumer benefits versus JOHNSON's Baby Lightoil Mist and JOHNSON's Baby Oil. Twenty micro liters of each of the three products were applied to a free area of skin and distributed into an area roughly the size of a 5 DM coin by rotating with the forefinger 15 times. Using a stopwatch, the skin was then assessed two minutes after the application was finished. A variety of attributes were evaluated, such as dispersal, sheen, residue, absorption, wateriness, greasiness, softness, and so forth.

The personal care composition of the invention scored higher versus the JOHNSON's products on the attributes of absorption, leaves less residue, less greasy film two minutes after application, and skin feel much dry.

It is understood that wile the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A personal care composition comprising at least one oil and at least one siloxane resin foam stabilizer, said composition being substantially free of surfactants and/or propellants, and being capable of foaming.

2. The composition of claim 1, wherein the siloxane resin foam stabilizer comprises monovalent trihydrocarbonsiloxy (M) groups of the formula R"₃SiO_{1/2} and tetrafunctional (Q) groups R"SiO_{4/2}, wherein R" denotes a monovalent alkyl group and the number ratio of M groups to Q groups is in the range 0.4:1 to 1.1:1.

3. The composition of claim 1 or 2, wherein the oil is selected from the group consisting of natural or synthetic oils from mineral, vegetable, or animal sources, fats, hydrocarbon oils, silicone oils, linear volatile polydialkylsiloxanes, in particular linear volatile polydimethylsiloxanes, cyclic volatile polydialkylsiloxanes, in particular cyclic volatile polydimethylsiloxanes, linear nonvolatile polydialkylsiloxanes, in particular linear nonvolatile polydimethylsiloxanes, polyalkyl siloxanes, polyaryl siloxanes, in particular polyphenylsiloxanes, polyalkylaryl siloxane copolymers, in particular polybenzyl siloxane copolymers, silicone resins, siloxy silicate polymers, fatty acid esters, fatty alcohols, fatty acids and mixtures thereof.

4. The composition of claim 3, wherein the oil comprises one or more vegetable oils.

5. The composition of any of claims 1 to 4 further comprising a cosmetically active agent selected from anti-aging agents, lightening agents, darkening agents, anti-acne agents, shine control agents, anti-microbial agents, anti-fungal agents, anti-bacterial agents, anti-inflammatory agents, anti-parasite agents, external analgesics, sunscreens, photoprotectors, antioxidants, keratolytic agents, moisturizers, nutrients, vitamins, energy enhancers, anti-perspiration agents, astringents, deodorants, hair removers, hair growth enhancing agents, hair growth delaying agents, firming agents, anti-callous agents, agents for skin conditioning, anti-cellulite agents, fluorides, odor-control agents, actives from natural sources, and mixtures thereof.

6. The composition of any of claims 1 to 5, further comprising at least one emollient and/or thickener.

7. The composition of any of claims 1 to 6, further comprising at least one volatile alkane, in particular a C₆- to C₁₈- volatile alkane, or mixtures thereof.

8. The composition of any of claims 1 to 7, further comprising at least one solubilizing agent for the siloxane resin foam stabilizer in the form of at least one fatty acid ester.

9. Use of the personal care composition of any of claims 1 to 8 in a skin care product selected from the group consisting of oil baths, shower preparations, face cleansers, body cleansers, make-up removers, and eye make-up removers.

10. Use of the personal care composition of any of claims 1 to 8 in a baby care product selected from the group consisting of baby toiletries, baby hair care products, baby skin care products, baby sun care products, baby washes, and baby cleansers.

11. Use of the personal care composition of any of claims 1 to 8 for the preparation of a skin care product selected from the group consisting of oil baths, shower preparations, face cleansers, body cleansers, make-up removers, and eye make-up removers.

12. Use of the personal care composition of any of claims 1 to 8 for the preparation of a baby care product selected from the group consisting of baby toiletries, baby hair care products, baby skin care products, baby sun care products, baby washes, and baby cleansers.

13. Use of the personal care composition of any of claims 1 to 8 to prepare compositions having reduced greasiness.

14. A method for reducing the greasiness of an oil-containing personal care composition, which comprises adding thereto a siloxane resin foam stabilizer, in particular to obtain a composition according to any of claims 1 to 8.

15. The method of claim 14, wherein the siloxane resin foam stabilizer comprises monovalent trihydrocarbonsiloxy (M) groups of the formula R"₃SiO_{1/2} and tetrafunctional (Q) groups R"SiO_{4/2}, wherein R" denotes a monovalent alkyl group and the number ratio of M groups to Q groups is in the range 0.4:1 to 1.1:1.

16. The method of claim 14 or 15, wherein the oil is selected from the group consisting of natural or synthetic oils from mineral, vegetable, or animal sources, fats, hydrocarbon oils, silicone oils, linear volatile polydimethylsiloxanes, cyclic volatile polydimethylsiloxanes, linear nonvolatile polydimethylsiloxanes, polyalkyl siloxanes, polyaryl siloxanes, polyalkylaryl siloxane copolymers, silicone resins, siloxy silicate polymers, fatty acid esters, fatty alcohols, fatty acids and mixtures thereof.

17. The method of claim 16, wherein the oil comprises one or more vegetable oils.

18. The method of any of claims 14 to 17 , wherein the personal care composition further comprises a cosmetically active agent selected from anti-aging agents, lightening agents, darkening agents, anti-acne agents, shine control agents, anti-microbial agents, anti-fungal agents, anti-bacterial agents, anti-inflammatory agents, anti-parasite agents, external analgesics, sunscreens, photoprotectors, antioxidants, keratolytic agents, moisturizers, nutrients, vitamins, energy enhancers, anti-perspiration agents, astringents, deodorants, hair removers, hair growth enhancing agents, hair growth delaying agents, firming agents, anti-callous agents, agents for skin conditioning, anti-cellulite agents, fluorides, odor-control agents, actives from natural sources, and mixtures thereof.

19. The method of any of claims 14 to 18, wherein the personal care composition further comprises at least one emollient and/or thickener.
